(19) **Europäisches Patentamt**
**European Patent Office**
**Office européen des brevets**

(11) **EP 2 526 864 B1**

(12) **EUROPEAN PATENT SPECIFICATION**

(45) Date of publication and mention
of the grant of the patent:
**02.08.2017 Bulletin 2017/31**

(51) Int Cl.:
***A61B 5/11*** *(2006.01)*

(21) Application number: **12161855.7**

(22) Date of filing: **28.03.2012**

(54) **Body movement detecting apparatus, method, computer program and computer-readable storage medium**

Vorrichtung, Verfahren, Computer-Programm und Medium zum Erkennen der Körperbewegung

Appareil, procédé, programme d'ordinateur et support lisible par un ordinateur pour la détection des mouvements du corps

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.05.2011 JP 2011117286**

(43) Date of publication of application:
**28.11.2012 Bulletin 2012/48**

(73) Proprietor: **Fujitsu Limited**
**Kawasaki-shi, Kanagawa 211-8588 (JP)**

(72) Inventors:
• **Tanaka, Masakiyo**
**Kawasaki-shi, Kanagawa 211-8588 (JP)**
• **Ohtani, Takeshi**
**Kawasaki-shi, Kanagawa 211-8588 (JP)**
• **Suzuki, Masanao**
**Kawasaki-shi, Kanagawa 211-8588 (JP)**

(74) Representative: **Stebbing, Timothy Charles**
**Haseltine Lake LLP**
**Lincoln House, 5th Floor**
**300 High Holborn**
**London WC1V 7JH (GB)**

(56) References cited:
WO-A1-2006/033104    WO-A1-2011/010384
WO-A2-2008/098943    JP-A- 2009 160 341
US-A1- 2004 034 285    US-A1- 2011 112 442

## Description

FIELD

The embodiments discussed herein are related to a body movement detecting apparatus, a body movement detecting method, and a non-transitory recording medium storing a body movement detecting program.

BACKGROUND

Body movement during sleep is known to be one of the characteristics in a human body utilized for measuring a sleeping status of a human being. For example, the low frequency of the presence of body movement may be defined as a deep sleeping status whereas the high frequency of the presence of body movement may be defined as a light sleeping status or an awakening status.

There is disclosed in the related art a technology of detecting such body movement utilizing a predetermined sensor. For example, there is disclosed a technology of detecting body movement utilizing an infrared ray sensor. In this technology, the infrared ray sensor is directed at a user such that body movement of the user is detected as fluctuated values of the infrared sensor.

Further, there is disclosed another technology of detecting body movement utilizing a pressure sensor. In this technology, the pressure sensor is set in a bed such that body movement of a user is detected as fluctuated values of the pressure sensor caused by the body movement.

In addition, there is disclosed another technology of detecting body movement utilizing an acceleration sensor. In this technology, the acceleration sensor is attached to an arm of a user such that body movement of the user is detected as fluctuated values of the acceleration sensor caused by the body movement.

Related-Art Document:

Patent Document 1: Japanese Laid-open Patent Publication No. 2007-289660
Patent Document 2: Japanese Laid-open Patent Publication No. 2008-301951
Non-Patent Document 1: NAKAYAMA, E. et al. "A Basic Study on Sleep-Wake Identification by Wrist Actigraph", Ishikawa Journal of Nursing, Vol. 3(2), 2006, Page 31-37

However, with the above related art technologies, expensive special-purpose sensors are utilized, and hence detecting the body movement may cost exceedingly high. Thus, a use of a low price sensor for detecting the body movement may be proposed. One example of such a low price sensor that is utilized for detecting the body movement may be a microphone. In this example, acoustic signals are input via the microphone and the acoustic signals indicating the volume above a predetermined level (i.e., predetermined loudness) may be detected as the body movement.

However, in this example, disturbance noise due to home electronic equipment and appliances or external noise may also be detected as the body movement, and hence, performance of the microphone may be adversely affect by such disturbance noise.

WO2011010384 (A1) discloses a sleep apnea syndrome examination device including an analysis unit that analyzes every unit time an acoustic signal for an examinee to cause at the sleeping time; a judgment unit that judges whether a characteristic sound or respiratory sound is contained in the unit time acoustic signal caused at the time when a sleep apnea syndrome patient recovers an respiration condition from an apnea condition, judges that a sleeping condition of the examinee is "the apnea condition" seen from the unit time acoustic signal in the case where the characteristic sound is contained in the unit time acoustic signal, judges that the sleeping condition of the examinee during the unit time is "the respiration present condition" in the case where the characteristic sound is not contained but the respiration sound is contained in the unit time acoustic signal, and judges that a sleeping condition of the examinee during the unit time is "the respiration absent condition" in the case where neither the characteristic sound nor the respiration sound is contained in the unit time acoustic signal; a storage unit that stores a sleeping condition of the examinee every unit time; and a detection unit that detects the apnea condition of the examinee in the case where a history of the examinee shifts at least from "the respiration non-existence condition" to "an apnea restoring condition".

US2011112442 (A1) discloses an apparatus including at least one sensor, configured to sense a physiological parameter of a subject and to sense large body movement of the subject, an output unit, and a control unit. The control unit is configured to monitor a condition of the subject by analyzing the physiological parameter and the sensed large body movement, and to drive the output unit to generate an alert upon detecting a deterioration of the monitored condition.

WO2006033104 (A1) discloses a system for detecting non-verbal acoustic energy generated by a subject is provided. The system includes a sensor mountable on or in a body region of the subject, the sensor being capable of sensing the non-verbal acoustic energy; and a processing unit being capable of processing the non-verbal acoustic energy sensed by the sensor and deriving an activity related signature therefrom, thereby enabling identification of a specific activity associated with the non-verbal acoustic energy.

WO2008098943 (A2) and intelligent alarm clock arrangement is adapted into an electric device like a mobile cell phone or a small personal computer (PDA) and utilizing the microphone, loudspeaker or other alarming device, memory unit, processor and timer feature thereof. The apparatus is located near to the sleeping subject so as to sample and analyze the statistical properties of the sound signals produced by the movements of the sleeping subject and to classify the sleep states according to the analyzed signals into a peaceful calm deep sleep, a light sleep with arousals and awake periods associated with the movements. An alarm wakes up the subject during a pre-programmed time window, if there are arousals, awake state and .movements present at that time to induce the awakening at a biologically advantageous instant.

SUMMARY

Accordingly, it is an object in one aspect of the invention to provide a body movement detecting apparatus according to claim 1, a method for detecting body movement according to claim 6, a body movement detecting computer program according to claim 7, and a computer-readable storage medium according to claim 8 that may be capable of detecting body movement with high accuracy utilizing acoustic properties of sound of the body movement.

BRIEF DESCRIPTION OF THE DRAWINGS

FIGS. 1A, 1B and 1C are diagrams illustrating a body movement sound generating model;
FIG. 2 is a block diagram illustrating an example of a body movement detecting apparatus according to a first embodiment;
FIG. 3 is a diagram illustrating an example of the frequency of sound generated by body movement;
FIGS. 4A and 4B are diagrams illustrating examples of electric power differences in predetermined frequency bandwidths;
FIG. 5 is a diagram illustrating the frequency of correlation values between the electric power differences;
FIGS. 6A and 6B are diagrams illustrating examples of electric power differences in two frequency bandwidths;
FIG. 7 is a diagram illustrating a process of detecting the body movement;
FIG. 8 is a diagram illustrating an example of the frequency spectrum of sound of running water;
FIG. 9 is a diagram illustrating characteristics of the running water sound;
FIG. 10 is a diagram illustrating an example of the frequency spectrum of electric train traveling noise;
FIG. 11 is a diagram illustrating characteristics of the electric train traveling noise;
FIG. 12 is a flowchart illustrating an example of a body movement detecting process according to the first embodiment;
FIG. 13 is a block diagram illustrating an example of a body movement detecting apparatus according to a second embodiment;
FIG. 14 is a diagram illustrating an interval of which a similarity value computing part computes correlation values;
FIG. 15 is a flowchart illustrating an example of a body movement detecting process according to the second embodiment; and
FIG. 16 is a block diagram illustrating an example of hardware of a mobile terminal apparatus.

DESCRIPTION OF EMBODIMENTS

First, characteristics of the body movement sound are described. The body movement sound is generated by friction-induced vibrations, such as friction caused in a sleeping mattress or clothes, transferred in the air while a user is sleeping. The characteristics of such a body movement sound known in the related art include small electric power differences occurring in numerous frequency bandwidths where the electric power differences are similar to one another between the frequency bandwidths. The reasons that these two characteristics represent the body movement sound are described below.

(BODY MOVEMENT SOUND GENERATING MODEL)

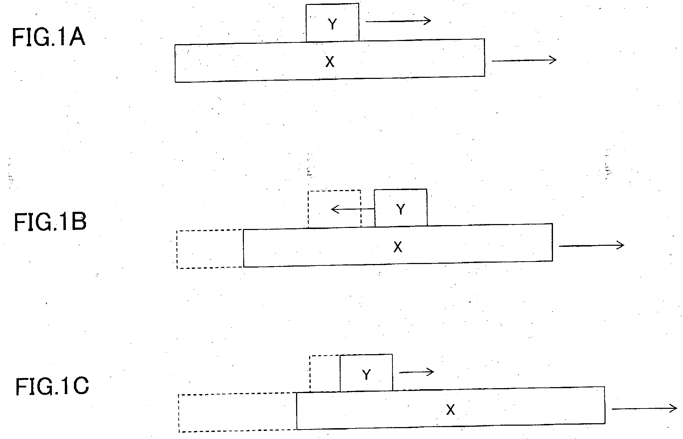

FIGS. 1A, 1B and 1C are diagrams illustrating a model of generating body movement sound (hereinafter called a "body movement sound generating model"). In FIGS. 1A to 1C, X represents a body of the user and Y represents fabric of clothes or a sleeping mattress. First, a case where X (i.e., the body of the user) moves in a direction of arrow in FIG. 1A is described.

As illustrated in FIG. 1A, when X moves in the direction of a corresponding arrow, Y also moves along with X due to the force of friction between X and Y. However, when the displacement of Y reaches or becomes greater than a predetermined amount, the force to move Y back to the original position (i.e., restoring force) becomes greater than the frictional force, and as a result, Y gradually moves back to the original position as illustrated in FIG. 1B. When the displacement of Y becomes less than the predetermined amount, the restoring force becomes less than the frictional force, and as a result, Y starts moving again along with X as illustrated in FIG. 1C.

Y is vibrated by the repeated movements of FIGS. 1B and 1C. The generated vibration is then transferred in the air to generate the body movement sound. Note that the frequency of the vibration of Y corresponds to the frequency of the body movement sound and hence, the amplitude of the vibration of Y represents the loudness of the body movement sound. The reasons are as follows.

(FREQUENCY OF BODY MOVEMENT SOUND)

The vibration frequency of Y (e.g., fabric) is determined based on the number of repeated cycles of the movements of FIGS. 1B and 1C. The following equation (1) is obtained by solving the equation of motion (see Kunieda, M., Practical use, Mechanical Vibration (in Japanese), 1990, Rikogakusha Publishing Co., Ltd:

http://www.mech-da.co.jp/mechnews/95-1/1995-1-1.html).

$$f = \frac{1}{2\pi} \sqrt{\frac{k}{m} - \left(\frac{\beta}{2m}\right)^2} \quad \cdots\cdots (1)$$

f: Frequency
m: Mass of Y
k: Restoring force of Y (i.e., spring constant)
$\beta$: Unique coefficient determined by coefficient of friction

Since soft raw materials formed of fabric, such as a mattress or clothes, acquire various values of k and $\beta$ based on shapes and movements of the soft raw materials, the entire mattress or clothes may seem to vibrate at various frequencies when computed by the equation (1). Thus, the body movement sound may have the frequency component of a wide bandwidth. Further, referring to the equation (1), the vibration frequency f may not depend on the velocity of the body (X) or the fabric (Y).

(AMPLITUDE OF BODY MOVEMENT SOUND)

As already mentioned in the frequency of body movement sound section above, the vibration frequency of the fabric obtained by the body movement is constant regardless of the velocity of the fabric. Thus, based upon the fact that the frequency of the body movement sound is constant regardless of the velocity of the fabric, the higher the velocity of the fabric, the wider the amplitude of the body movement sound will be.

Since the fabric moves along the body movement due to the friction generated between the body movement and the fabric, the velocity of the fabric movement may be determined based on the velocity of the body movement. When f represents the vibration frequency and V represents the velocity of the body movement, the amplitude A of the fabric vibration may be computed based on the following equation (2).

$$A = \frac{V}{2f} \quad \cdots\cdots (2)$$

As illustrated in the equation (2), the amplitude of the fabric movement (the loudness of the body movement sound) is determined in proportion to the velocity of the body movement.

(CHARACTERISTICS OF BODY MOVEMENT SOUND)

To summarize the above, the body movement sound is generated by the vibration due to the friction of the fabric of the mattress or clothes along with with the body movement. The frequency of the fabric sound may be constant regardless of the velocity of the body movement and may vary with the friction coefficients. The amplitude of the fabric sound (i.e., the loudness of the body movement sound) is in proportion to the velocity of the body movement.

Note that the velocity change in the body movement may seem to be small, in general, excluding the velocity changes obtained immediate before and after the outset of the body movement. Further, the fact that the velocity change in the body movement is small indicates that the amplitude change in the fabric sound is small, and similarly indicates that the electric power difference at each of the frequencies of the body movement sound is small.

Further, although the velocity change in the body movement is small, the velocity of the body movement varies. Consequently, the amplitude of the fabric sound changes along with the velocity change of the body movement. Thus, similar electric power differences may be obtained in the frequency bandwidths of the body movement sound.

Accordingly, the body movement sound may have two characteristics, that is, the small electric power differences being obtained in the numerous frequency bandwidths, and the similar electric power differences being obtained in the frequency bandwidths. Preferred embodiments will be described below with reference to the accompanying drawings.

[FIRST EMBODIMENT]

[CONFIGURATION]

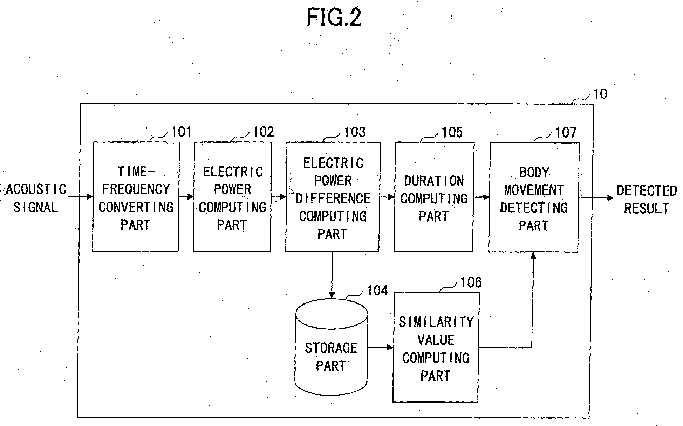

FIG. 2 is a block diagram illustrating an example of a body movement detecting apparatus according to a first embodiment. A body movement detecting apparatus 10 includes a time-frequency converting part 101, an electric power computing part 102, an electric power difference computing part 103, a storage part 104, a duration computing part 105, a similarity value computing part 106 and a body movement detecting part 107.

The time-frequency converting part 101 is configured to receive an acoustic signal input by an input device such as a microphone, and perform time-frequency conversion on the received acoustic signal. As examples of the time-frequency conversion, the discrete-time Fourier transform (DTFT) and the wavelet transform may be given. In this embodiment, the discrete-time Fourier transform (DTFT) is utilized as the example of the time-frequency conversion.

Specifically, the time-frequency converting part 101 converts the received acoustic signal into a signal in a frequency domain (i.e., the frequency component) based on the following equation (3).

$$S(n,k) = \sum_{j=0}^{K-1} s(n,j) \exp(-\frac{2\pi i}{K} jk) \quad (k = 0,1...K-1) \quad .......... (3)$$

S(n,k): Fourier transformation result in kth frequency bandwidth of signal array s(n,j)

The score (frame length) of the discrete Fourier transform may, for example, be determined as 256 points (16 ms) at the acoustic signal sampling frequency of 16 kHz. The converted S (n, k) is output to the electric power computing part 102.

The converted S (n, k) may be expressed by two separate parts, namely, a real part and an imaginary part, as expressed by the following equation (4).

$$S(n,k) = S\_re(n,k) + iS\_im(n,k) \quad .......... (4)$$

S_re(n,k): Real part of S(n,k)
S_im(n,k): Imaginary part of S(n,k)

The electric power computing part 102 is configured to compute the power spectrum for the nth frame and the kth frequency bandwidth based on the following equation (5).

$$pow(n,k) = \|S\_re(n,k)\|^2 + \|S\_im(n,k)\|^2 \quad (k = 0,1...K-1) \quad .......... (5)$$

pow(n,k): Power spectrum in nth frame and kth frequency bandwidth (Electric power)

The electric power computing part 102 outputs to the electric power difference computing part 103 the power pow (n, k) in each of the frequency bandwidths computed based on the sum of squares of the real part and the imaginary part of the frequency component.

The electric power difference computing part 103 is configured to acquire the electric power pow(n,k) and compute as the difference between the power pow(n-1,k) in a preceding frame n-1 and the electric power pow(n,k) in a current frame n for each of the frequency bandwidths as an electric power difference value based on the following equation (6). Note that the electric power difference value is hereinafter referred to as an "electric power difference".

$$pow\_diff(n,k) = pow(n,k) - pow(n-1,k) \qquad \cdots\cdots\cdots (6)$$

pow_diff (n,k): Electric power difference for nth frame and kth frequency bandwidth

The electric power difference computing part 103 outputs the computed electric power difference pow_diff(n, k) to the duration computing part 105. The electric power difference computing part 103 also stores the computed electric power difference pow_diff(n,k) in the storage part 104.

The electric power difference computing part 103 also stores the computed electric power difference pow_diff(n, k) in the storage part 104. The storage part 104 may be configured to store the electric power difference for the latest predetermined duration based on the capacity of a memory area.

The duration computing part 105 adds one frame to the duration if the number of frequency bandwidths each exhibiting the absolute value of the electric power difference less than a threshold TH_POW is equal to or greater than a threshold TH_NUM. The duration computing part 105 resets (clears) the duration if the number of frequency bandwidths each exhibiting the absolute value of the electric power difference less than a threshold TH_POW is less than the threshold TH_NUM.

The aforementioned processes are expressed by the following equations (7) to (9).

$$pow\_diff\_func(n,k) = \begin{cases} 1 & (|pow\_diff(n,k)| < TH\_POW) \\ 0 & (otherwise) \end{cases} \qquad \cdots\cdots (7)$$

$$num\_pow\_diff(n) = \sum_{k=0}^{K-1} \{pow\_diff\_func(n,k)\} \qquad \cdots\cdots (8)$$

$$duration(n) = \begin{cases} duration(n-1)+1 & (num\_pow\_diff(n) \geq TH\_NUM) \\ 0 & (otherwise) \end{cases} \qquad \cdots\cdots (9)$$

pow_diff_func(n,k): Function to return "1" when the absolute value of the electric power difference is less than the threshold TH_POW, otherwise to return "0" num_powdiff(n): Number of frequency bandwidths each exhibiting the absolute value of the electric power difference less than the threshold TH_POW

duration (n): Frame duration in which the number of frequency bandwidths each exhibiting the absolute value of the electric power difference less than the threshold TH_POW is greater than the threshold TH_NUM

For example, the threshold TH_POW may be set as 3dB and the threshold TH_NUM may be set as half (1/2) of the number of frequency bandwidths. For example, when the number of frequency bandwidths is 128, the threshold TH_NUM is 64. The duration computing part 105 outputs the computed duration to the body movement detecting part 107.

The similarity value computing part 106 acquires the electric power difference for a predetermined duration from the storage part 104 and computes a value indicating similarity (hereinafter referred to as a "similarity value") between the electric power differences of the frequency bandwidths. The predetermined duration may, for example, be 2 s (e.g., 125 frames when the sampling frequency is 16 kHz and one frame is 16 ms). This is based on the fact that the body movement is generally represented by 2 s or above.

The similarity value computing part 106 is configured to use the sum of correlation values or the value computed based on a signum function of the electric power difference as the similarity value.

[SUM OF CORRELATION VALUES]

When the sum of correlation values is employed as the similarity value, the similarity value computing part 106 acquires the sum of correlation values of all the frequency bandwidths based on the following equations (10) and (11).

$$corr(n-1,k,l) = \frac{\sum_{m=n-1-M}^{n-1}\{(pow\_diff(m,k)-pow\_diff\_ave(k))\times(pow\_diff(m,l)-pow\_diff\_ave(l)\}}{\sqrt{\sum_{m=n-1-M}^{n-1}(pow\_diff(m,k)-pow\_diff\_ave(k))^2 \times \sum_{m=n-1-M}^{n-1}(pow\_diff(m,l)-pow\_diff\_ave(l))^2}} \qquad \cdots (10)$$

$$corr\_all(n-1) = \sum_{k=0}^{K-1}\sum_{l=k+1}^{K-1} corr(n-1,k,l) \qquad \cdots (11)$$

pow_diff_ave(k): Mean (average) of electric power differences
corr(n-1,k,l): Correlation between electric power differences in frequency bandwidths k and 1
corr_all(n-1): Sum of correlation values of all the frequency bandwidths

The similarity value computing part 106 may compute the sum of correlation values first, then divide the obtained sum of correlation values corr_all(n-1) by the number of combinations of all the frequency bandwidths, and finally normalize the computed result by values of 0 or 1. Hereinafter, a further description is given based on the assumption that the sum of correlation values corr_all(n-1) is normalized. The similarity value computing part 106 outputs the computed sum of correlation values corr_all(n-1) as the similarity value to the body movement detecting part 107.

(SIGNUM FUNCTION)

Based upon the fact that the electric power differences are similar in numerous frequency bandwidths, the signs (i.e., positive + or negative -) of the values of the electric power differences may be the same in the numerous frequency bandwidths. For example, whether the electric power is decreased or increased is the same in the numerous frequency bandwidths.

Accordingly, the following parameters utilizing the signum function of the electric power differences are applied as the similarity values of the electric power differences in the frequency bandwidths. When the signum function of the electric power differences is used, the similarity value computing part 106 computes the parameters of the signum function of the electric power differences based on the following equations (12) to (15).

$$sign(x) = \begin{cases} 1 & (x > 0) \\ 0 & (otherwise) \end{cases} \qquad \cdots (12)$$

$$powdiff\_sign\_p(n) = \sum_{k=1}^{K} sign\{pow\_diff(n,k)\} \qquad \cdots (13)$$

$$powdiff\_sign\_m(n) = K - powdiff\_sign\_p(n) \qquad \cdots (14)$$

$$powdiff\_sign(n) = max\{powdiff\_sign\_p(n), powdiff\_sign\_m(n)\} \qquad \cdots (15)$$

sign(x): Function to return "1" when x is a positive (+) value, otherwise to return "0"

powdiff_sign_p(n): Number of frequency bandwidths exhibiting electric power difference of a positive value at nth frame

powdiff_sign_m(n): Number of frequency bandwidths exhibiting electric power difference of a 0 value or less (a negative value) at nth frame

powdiff_sign (n): one of values being greater than the other between powdiff_sign_p(n) and powdiff_sign_m(n)

The similarity value computing part 106 adds one frame to a duration having a similar electric power difference (hereinafter referred to as "similarity duration") if the powdiff_sign(n) is equal to or greater than a threshold TH_SIGN. For example, the threshold TH_SIGN is determined as 80 when the frequency bandwidth is 128. The similarity value computing part 106 outputs the similarity duration to the body movement detecting part 107 as the similarity value.

The body movement detecting part 107 detects the body movement based on the duration computed by the duration computing part 105 and the similarity value (i.e., similarity duration) computed by the similarity value computing part 106.

(SUM OF CORRELATION VALUES APPLIED AS SIMILARITY VALUE)

For example, when the duration is equal to or greater than a threshold TH_TIME and the sum of correlation values is equal to or greater than a threshold TH_COR, the body movement detecting part 107 determines that the body movement is present in the corresponding duration, and hence, the body movement detecting part 107 detects the body movement. The threshold TH_TIME may be set as a value of 2 s or above and the threshold TH_COR may be set as 0.5. The electric power difference of the body movement sound frequently appears at the correlation value of 0.5 or above. Hence, if the sum of correlation values after the normalization is 0.5 or above, it may be possible to determine that the body movement is present.

(SIMILARITY DURATION APPLIED AS SIMILARITY VALUE)

For example, when the duration and the similarity duration are equal to or greater than the threshold TH_TIME, the body movement detecting part 107 determines that the body movement is present in the corresponding duration, and hence, the body movement detecting part 107 detects the body movement.

Note that when the body movement detecting part 107 detects the body movement, the body movement detecting part 107 may further be configured to record time at the outset of the body movement and also time at the end of the body movement in storage such as a memory. The recorded times at the outset and the end of the body movement may be utilized for obtaining a sleeping status of the user.

[EXAMPLE]

Next, the aforementioned processes are described with reference to accompanying drawings by way of a specific example in which the sum of correlation values is applied as the similarity value.

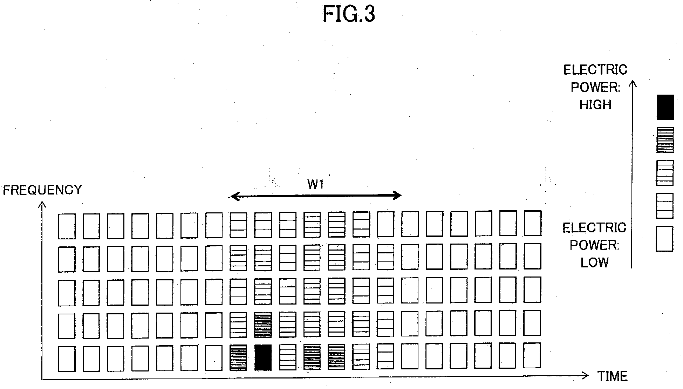

FIG. 3 is a diagram illustrating an example of the frequency of body movement sound. When the body movement is present in a range indicated by W1 in FIG. 3, the time-frequency converting part 101 converts the body movement sound into the frequency component, which increases the electric power in each of the frequency bandwidths. Note that the generation of the body movement sound having the characteristics illustrated in FIG. 3 lasts for a few to several tens s.

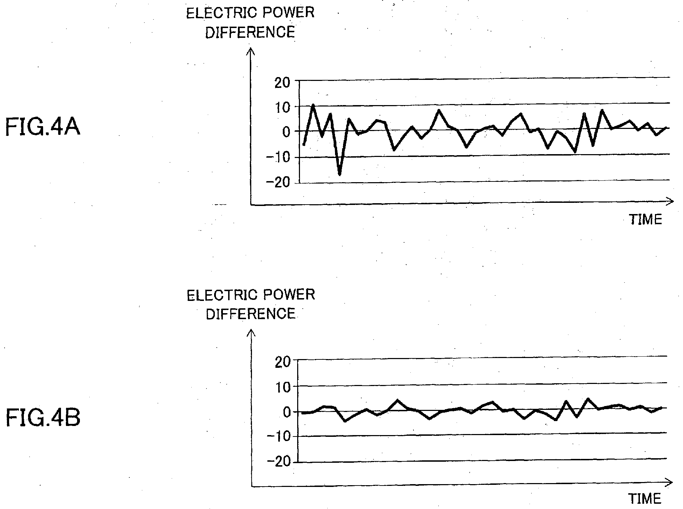

The electric power difference computing part 103 computes the electric power difference corresponding to the electric power computed by the electric power computing part 102. FIGS. 4A and 4B are diagrams illustrating examples of the electric power differences in predetermined frequency bandwidths. FIG. 4A illustrates the example of the electric power difference for an audio sound and FIG. 4B illustrates the example of the electric power difference for the body movement sound.

The electric power difference for the audio sound is large as illustrated in FIG. 4A, whereas the electric power difference for the body movement sound is small as illustrated in FIG. 4B. As mentioned earlier, the small electric power difference is one of the characteristics of the body movement sound. Thus, if the acoustic signal exhibits a large number of the frequency bandwidths each exhibiting the small electric power difference, it is probable that the acoustic signal is the body movement sound.

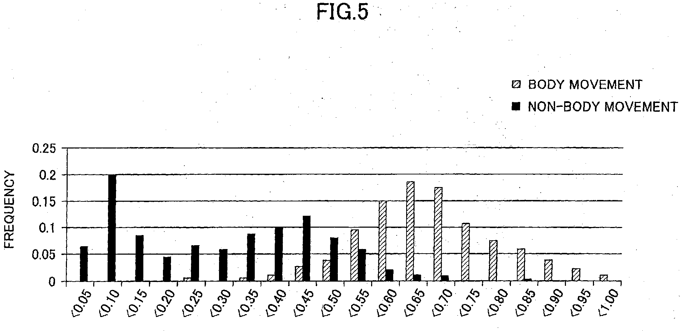

Next, the similarity value computing part 106 computes the sum of correlation values between the electric power differences. FIG. 5 is a diagram illustrating the frequency of correlation values between the electric power differences. In FIG. 5, the frequency of the correlation values indicates the proportion of the correlation values. As illustrated in FIG. 5, the non-body movement frequently appears at the correlation value of 0.5 or below whereas the body movement frequently appears at the correlation value of 0.5 or above.

As described above, in the body movement sound, the correlation values are distributed close to 0.5 or above.

Thus, if the normalized sum of correlation values is 0.5 or above, it is probable that the normalized values represent the body movement sound.

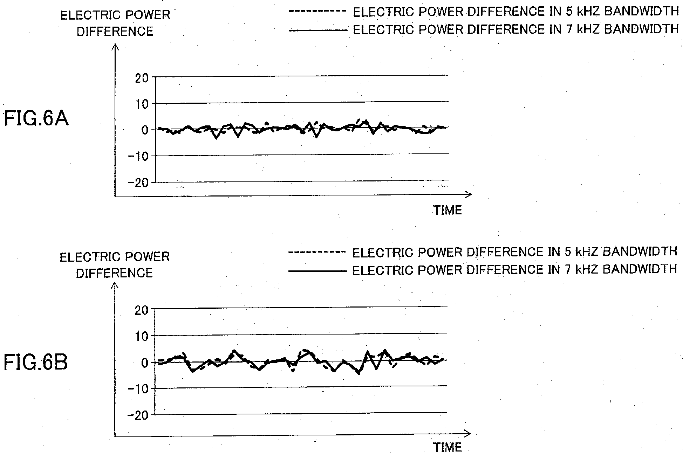

FIGS. 6A and 6B are diagrams illustrating an example of the electric power differences in two frequency bandwidths. FIG. 6A illustrates examples of background noise electric power differences in 5 kHz and 7 kHz. As illustrated in FIG. 6A, the background noise electric power difference for 5 kHz and the background noise electric power difference for 7 kHz are not similar, and hence, there is no correlation between these two electric power differences.

FIG. 6B illustrates example of body movement sound electric power differences in 5 kHz and 7 kHz. As illustrated in FIG. 6B, the body movement sound electric power difference for 5 kHz and the body movement sound electric power difference for 7 kHz are similar, and hence, there is a correlation between these two electric power differences. As mentioned earlier, the similarity between the electric power differences is one of the characteristics of the body movement sound.

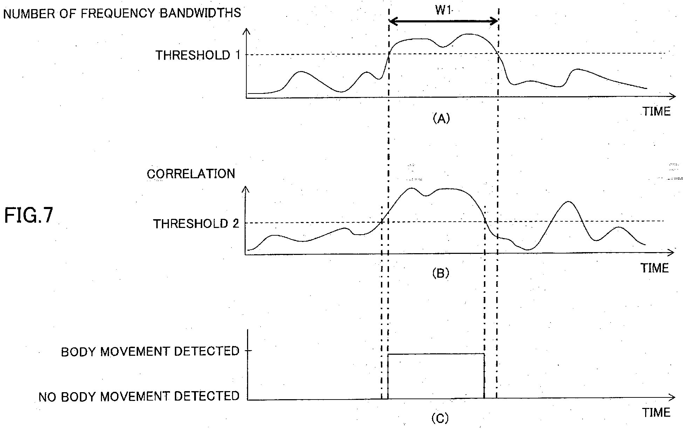

FIG. 7 is a diagram illustrating the detection of the body movement sound. (A) of FIG. 7 illustrates the number of frequency bandwidths each exhibiting the small electric power difference. In (A) of FIG. 7, the threshold 1 represents the threshold TH_NUM. As illustrated in (A) of FIG. 7, the frequency bandwidth is equal to or greater than the threshold 1 (threshold TH_NUM) within a range W1 where the body movement sound is generated. (B) of FIG. 7 illustrates the sum (i.e., the normalized sum) of correlation values between the electric power differences in the frequency bandwidths. In (B) of FIG. 7, the threshold 2 represents the threshold TH_COR. As illustrated in (B) of FIG. 7, the sum of correlation values between the electric power differences is equal to or greater than the threshold 2 (threshold TH_COR) within the range W1 where the body movement is present.

(C) of FIG. 7 illustrates the detected body movement. As illustrated in (C) of FIG. 7, the body movement detecting part 107 detects the body movement when the duration, in which the number of frequency bandwidths is equal to or greater than the threshold 1 and the sum of correlation values is equal to or greater than the threshold 2, is equal to or greater than the threshold TH_TIME.

Thus, the body movement detecting part 107 detects the body movement based on the duration in which the number of frequency bandwidths each exhibiting the small electric power difference is equal to or greater than the threshold, and a value indicating the similarity between the electric power differences in the frequency bandwidths.

Note that the reasons for utilizing the two characteristics, which are the small electric power difference and the similar electric power differences in the frequency bandwidths, in detecting the body movement are described below. If the body movement is detected by utilizing only one of the above characteristics, it is highly probable to erroneously detect other signals.

That is, air conditioners and background noise also exhibit small electric power differences. Hence, signals of such air conditioners and background noise may be erroneously detected if only the characteristic of the small electric power difference is utilized for detecting the body movement. Similarly, musical tones or vehicle travelling noise also exhibit small electric power differences. Hence, signals of such musical tones or vehicle travelling noise may be erroneously detected if only the characteristic of the similar electric power differences being obtained in the frequency bandwidths is utilized for detecting the body movement.

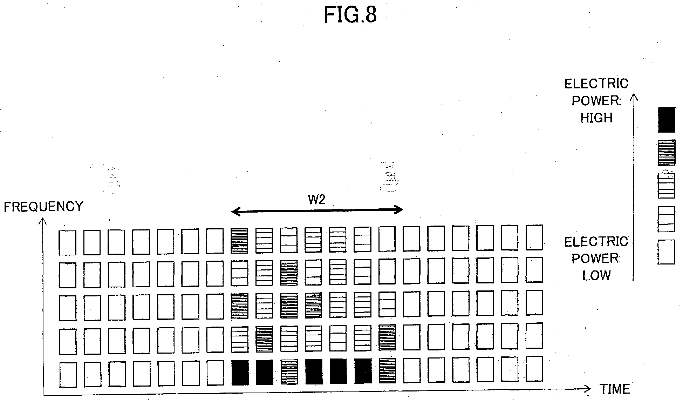

FIG. 8 is a diagram illustrating an example of the frequency spectrum of the sound of running water. In FIG. 8, the running water sound is generated within a range W2.

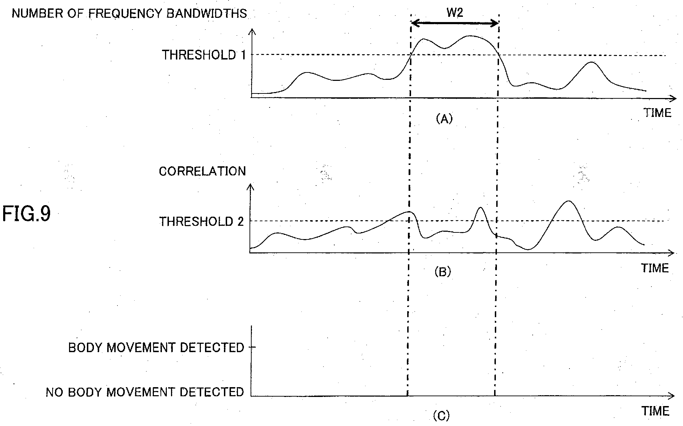

FIG. 9 is a diagram illustrating the characteristics of the running water sound. (A) of FIG. 9 illustrates the number of frequency bandwidths each exhibiting the small electric power difference. In (A) of FIG. 9, the threshold 1 represents the threshold TH_NUM. As illustrated in (A) of FIG.9, the number of frequency bandwidths is equal to or greater than the threshold 1 (threshold TH_NUM) within the range W2 where the running water sound is generated.

(B) of FIG. 9 illustrates the sum (i.e., the normalized sum) of correlation values between the electric power differences in the frequency bandwidths. In (B) of FIG. 9, the threshold 2 represents the threshold TH_COR. As illustrated in (B) of FIG. 9, the sum of correlation values between the electric power differences is not necessarily equal to or greater than the threshold 2 (threshold TH_COR) within a entire range W2 since the running water sound is generated.

(C) of FIG. 9 illustrates a result of the detected body movement. As illustrated in (C) of FIG. 9, the body movement detecting part 107 does not detect the body movement. This is because the duration, in which the number of frequency bandwidths is equal to or greater than the threshold 1 and the sum of correlation values is equal to or greater than the threshold 2, is less than the threshold TH_TIME.

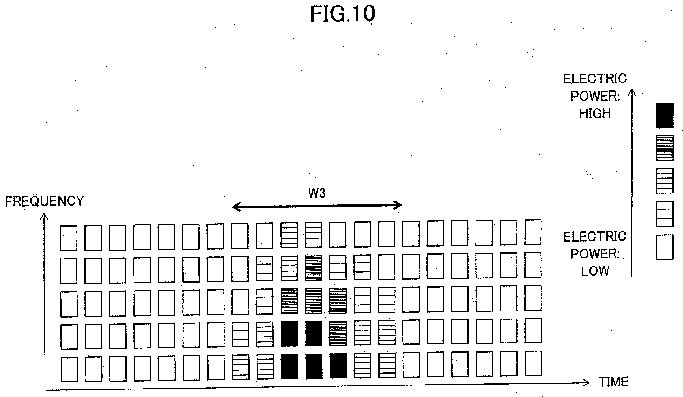

FIG. 10 is a diagram illustrating an example of the frequency spectrum of the electric train traveling noise. In FIG. 10, the electric train traveling noise is generated in a range W3.

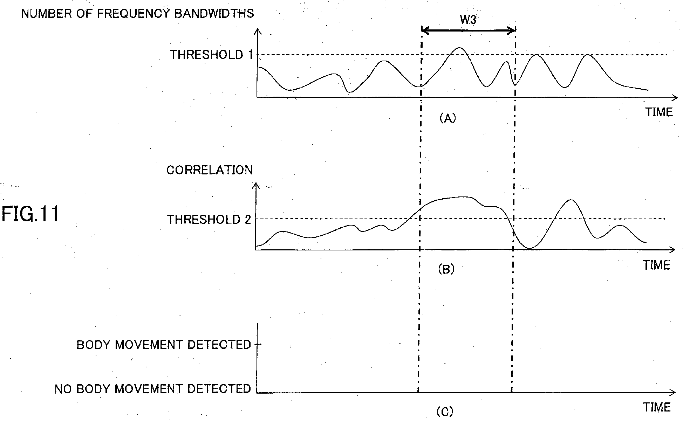

FIG. 11 is a diagram illustrating characteristics of the electric train traveling noise. (A) of FIG. 11 illustrates the number of frequency bandwidths each exhibiting the small electric power difference. In (A) of FIG. 11, the threshold 1 represents the threshold TH_NUM. As illustrated in (A) of FIG. 11, the number of frequency bandwidths is not necessarily equal to or greater than the threshold 1 within the entire range W3 where the electric train traveling noise is generated.

(B) of FIG. 11 illustrates the sum (i.e., the normalized sum) of correlation values between the electric power differences in the frequency bandwidths. In (B) of FIG. 11, the threshold 2 represents the threshold TH_COR. As illustrated in (B) of FIG. 11, the sum of correlation values of the electric power differences is equal to or greater than the threshold 2 (threshold TH_COR) within the range W3 where the electric train traveling noise is generated.

(C) of FIG. 11 illustrates a result of the detected body movement. As illustrated in (C) of FIG. 11, the body movement detecting part 107 does not detect the body movement. This is because the duration, in which the number of frequency bandwidths is equal to or greater than the threshold 1 and the sum of correlation values is equal to or greater than the threshold 2, is less than the threshold TH_TIME.

As described above, the body movement detecting apparatus 10 according to the first embodiment may be capable of detecting the body movement with high accuracy by utilizing the two characteristics of the body movement sound.

[OPERATION]

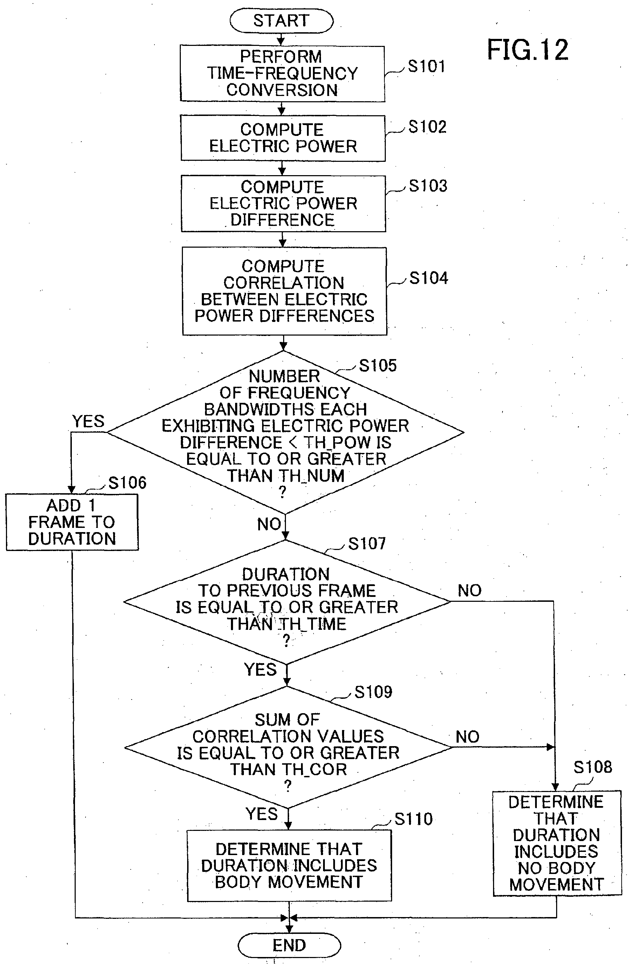

Next, operations of the body movement detecting apparatus 10 according to the first embodiment are described below. FIG. 12 is a flowchart illustrating an example of a body movement detecting process in the body movement detecting apparatus 10 according to the first embodiment. Specifically, FIG. 12 represents a process flow for each of the frames.

In step S101, the time-frequency converting part 101 receives an acoustic signal input by an input device such as a microphone, and converts the received acoustic signal into a frequency component to generate a frequency domain signal.

In step S102, the electric power computing part 102 computes the electric power based on the frequency domain signal acquired from the time-frequency converting part 101.

In step S103, the electric power difference computing part 103 computes difference of the electric power computed by the electric power computing part 102.

In step S104, the similarity value computing part 106 computes the sum of correlation values in the respective frequency bandwidths by utilizing the electric power differences computed by the electric power difference computing part 103. The sum of correlation values is normalized.

In step S105, the duration computing part 105 determines whether the number of frequency bandwidths each exhibiting the electric power difference less than the threshold TH_POW is equal to or greater than the threshold TH_NUM. If the number of frequency bandwidths is equal to or greater than the threshold TH_NUM ("YES" in step S105), step S106 is subsequently processed. If, on the other hand, the number of frequency bandwidths is less than the threshold TH_NUM ("NO" in step S105), step S107 is subsequently processed.

In step S106, the duration computing part 105 adds one frame to the duration and terminates the process of this frame.

In step S107, the body movement detecting part 107 determines whether the duration up to a previous frame is equal to or greater than the threshold TH_TIME. If the duration is equal to or greater than the threshold TH_TIME ("YES" in step S107), step S109 is subsequently processed. If, on the other hand, the duration is less than the threshold TH_TIME ("NO" in step S107), step S108 is subsequently processed.

In step S108, the body movement detecting part 107 determines that the body movement is not present in that duration (i.e., the duration includes no body movement) and terminates the process of this frame.

In step S109, the body movement detecting part 107 determines whether the normalized sum of correlation values is equal to or greater than the threshold TH_COR. If the normalized sum is equal to or greater than the threshold TH_COR ("YES" in step S109), step S110 is subsequently processed. If, on the other hand, the normalized sum is less than the threshold TH_COR ("NO" in step S109), step S108 is subsequently processed.

In step S110, the body movement detecting part 110 determines that the body movement is present in the corresponding duration (i.e., the duration includes body movement) and terminates the process of this frame.

As described above, the body movement detecting apparatus 10 according to the first embodiment may be capable of detecting body movement with high accuracy by utilizing the acoustic characteristics of the body movement sound. Further, the body movement detecting apparatus 10 according to the first embodiment may be capable of detecting body movement without utilizing expensive special-purpose sensors. In addition, the body movement detecting apparatus 10 according to the first embodiment may be simply placed at a position where the body movement sound is picked up or input and hence there are no positional limitations for placing the body movement detecting apparatus 10.

[SECOND EMBODIMENT]

Next, a body movement detecting apparatus 20 according to a second embodiment is described below. The body movement detecting apparatus 20 according to the second embodiment is a power saving model which is realized by computing the similarity value for identifying the similarity between the electric power differences when necessary.

[CONFIGURATION]

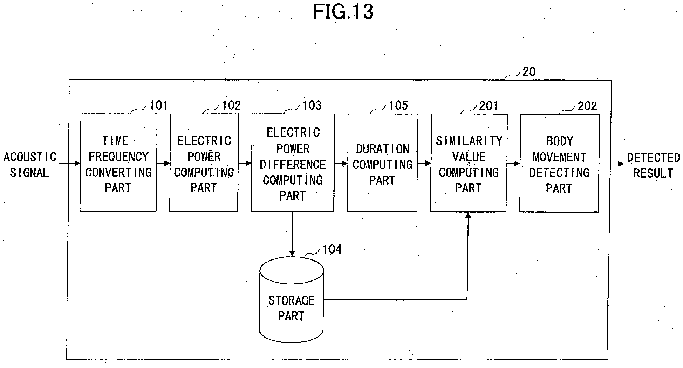

FIG. 13 is a block diagram illustrating an example of a configuration of a body movement detecting apparatus 20 according to the second embodiment. In the configuration illustrated in FIG. 13, components similar to those illustrated in FIG. 2 are provided with the same reference numerals and duplicated descriptions are omitted here.

As illustrated in FIG. 13, a similarity value computing part 201 acquires from the duration computing part 105 a duration in which the number of frequency bandwidths each exhibiting small electric power difference is equal to or greater than the threshold TH_NUM. In this case, when the duration is less than the threshold TH_NUM, the body movement detecting part 201 determines whether the duration up to a previous frame is equal to or greater than the threshold TH_TIME.

When the duration is less than the threshold TH_TIME, the similarity value computing part 201 reports that result to a body movement detecting part 202. When the duration is equal to or greater than the threshold TH_TIME, the similarity value computing part 201 computes a similarity value. In the second embodiment, the sum of correlation values between the electric power differences is utilized as the similarity value. Note that the similarity value computing part 201 may normalize the sum of correlation values in advance.

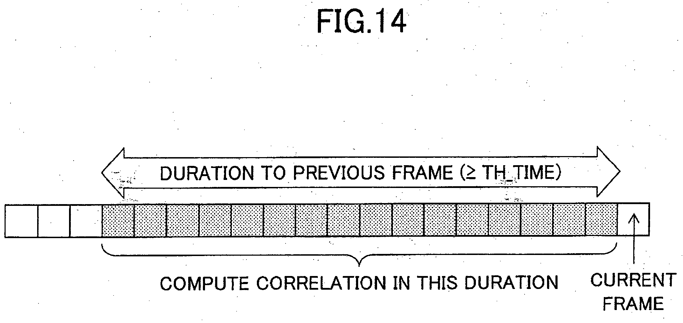

FIG. 14 is a diagram illustrating an interval of which the similarity value computing part 201 computes correlation values. As illustrated in FIG. 14, when the duration up to the previous frame is equal to or greater than the threshold TH_TIME, the similarity value computing part 201 computes a sum of correlation values between the electric power differences corresponding to frames of that interval (i.e., the duration up to the previous frame).

Thus, when the duration up to the previous frame is less than the threshold TH_TIME, the similarity value computing part 201 does not compute the sum of correlation values between the electric power differences. As a result, the body movement detecting apparatus 20 according to the second embodiment may save electric energy when detecting the body movement in this fashion. The similarity value computing part 201 outputs the computed sum of correlation values to the body movement detecting part 202.

The body movement detecting part 202 determines whether the sum of correlation values acquired from the similarity value computing part 201 is equal to or greater than the threshold TH_COR. If the sum of correlation values acquired from the similarity value computing part 201 is equal to or greater than the threshold TH_COR, the body movement detecting part 202 determines that the body movement is present in that duration (i.e., interval). That is, the duration includes the body movement. If, on the other hand, the sum of correlation values acquired from the similarity value computing part 201 is less than the threshold TH_COR, the body movement detecting part 202 determines that the body movement is not present in that duration (i.e., interval). That is, the duration includes no body movement. Further, when the body movement detecting part 202 receives from the body movement detecting part 201 a report indicating that the duration is less than the threshold TH_TIME, the body movement detecting part 202 determines that the duration includes no body movement.

Note that when the body movement detecting part 202 detects the body movement in a manner similar to that in the body movement detecting apparatus 10 according to the first embodiment, the body movement detecting part 202 may store a detected time at which the body movement is detected in a storage such as a memory. The recorded time at which the body movement is detected may be utilized for obtaining a sleeping status of the user.

[OPERATION]

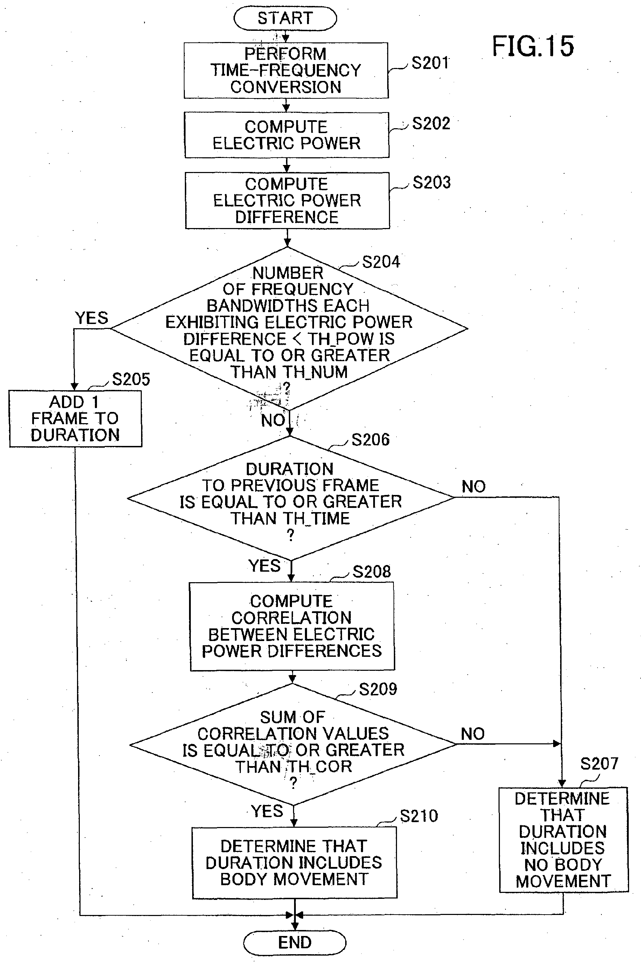

Next, operations of the body movement detecting apparatus 20 according to the second embodiment are described below. FIG. 15 is a flowchart illustrating an example of a body movement detecting process in the body movement detecting apparatus 20 according to the second embodiment. Specifically, FIG. 15 represents a process flow for each of the frames.

In step S201, the time-frequency converting part 101 receives an acoustic signal input by an input device such as a microphone, and converts the received acoustic signal into a frequency component to generate a frequency domain signal.

In step S202, the electric power computing part 102 computes the electric power based on the frequency domain signal acquired from the time-frequency converting part 101.

In step S203, the electric power difference computing part 103 computes difference of the electric power computed by the electric power computing part 102.

In step S204, the duration computing part 105 determines whether the number of frequency bandwidths each exhibiting the electric power difference less than the threshold TH_POW is equal to or greater than the threshold TH_NUM. If the number of frequency bandwidths is equal to or greater than the threshold TH_NUM ("YES" in step S204), step S205 is subsequently processed. If, on the other hand, the number of frequency bandwidths is less than the threshold TH_NUM ("NO" in step S204), step S206 is subsequently processed.

In step S205, the duration computing part 105 adds one frame to the duration and terminates the process of this frame.

In step S206, the similarity value computing part 201 determines whether the duration up to a previous frame is equal to or greater than the threshold TH_TIME. If the duration is equal to or greater than the threshold TH_TIME ("YES" in step S206), step S208 is subsequently processed. If, on the other hand, the duration is less than the threshold TH_TIME ("NO" in step S206), step S207 is subsequently processed.

In step S207, the body movement detecting part 202 receives from the similarity value computing part 201 a report indicating that the duration is less than the threshold TH_TIME, so that the body movement detecting part 202 determines that the body movement is not present in that duration (i.e., the duration includes no body movement) and terminates the process of this frame. Similarly, in step S207, the body movement detecting part 202 receives from the similarity value computing part 201 a report indicating that the sum of correlation values is less than the threshold TH_COR, so that the body movement detecting part 202 determines that the body movement is not present in that duration (i.e., the duration includes no body movement) and terminates the process of this frame.

In step S208, the similarity value computing part 201 computes the sum of correlation values in the respective frequency bandwidths by utilizing the electric power differences computed by the electric power difference computing part 103. The sum of correlation values is normalized.

In step S209, the body movement detecting part 202 determines whether the normalized sum of correlation values is equal to or greater than the threshold TH_COR. If the normalized sum is equal to or greater than the threshold TH_COR ("YES" in step S209), step S210 is subsequently processed. If, on the other hand, the normalized sum is less than the threshold TH_COR ("NO" in step S209), step S207 is subsequently processed.

In step S210, the body movement detecting part 202 determines that the body movement is present in that duration (i.e., the duration includes body movement) and terminates the process of this frame.

As described above, the body movement detecting apparatus 20 according to the second embodiment may further save the electric energy while maintaining the effect obtained in the first embodiment.

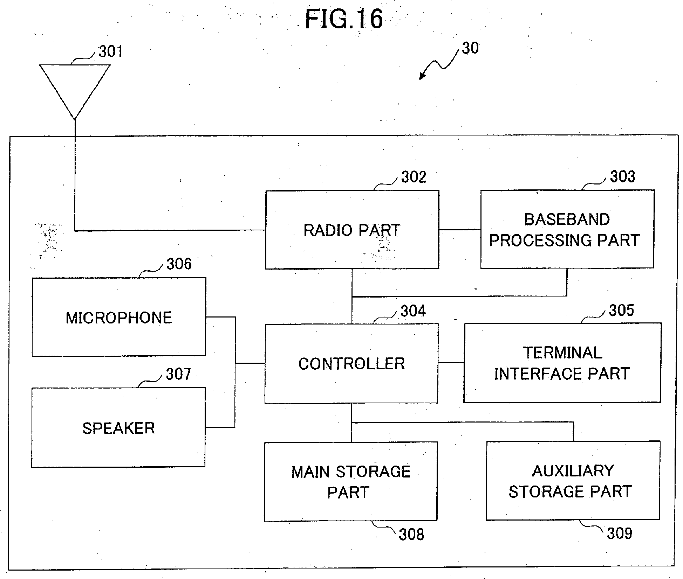

Next, hardware of a mobile terminal apparatus 30 including the body movement detecting apparatuses 10 and 20 illustrated according to the embodiments is described below. FIG. 16 is a block diagram illustrating an example of hardware of the mobile terminal apparatus 30. As illustrated in FIG. 16, the mobile terminal apparatus 30 includes an antenna 301, a radio part 302, a baseband processing part 303, a controller 304, a terminal interface part 305, a microphone 306, a speaker 307, a main storage part 308 and an auxiliary storage part 309.

The antenna 301 is configured to transmit a radio signal amplified by a transmission amplifier and receive a radio signal from a base station. The radio part 302 is configured to perform D/A conversion on the transmitted digital signal diffused by the baseband processing part 303 and perform quadrature modulation on the modulated signal to amplify the modulated signal by a power amplifier. The radio part 302 is configured to amplify the received radio signal and perform A/D conversion on the amplified signal to transmit A/D converted signal to the baseband processing part 303.

The baseband processing part 303 is configured to perform base band processing including attaching a transmitting data error-correcting code, modulating data, spreading modulation, dispreading the received signal, determining a signal receiving environment, determining respective thresholds of the channel signals and decoding the error-correcting code.

The controller 304 is configured to perform wireless control such as transmitting and receiving a control signal. The controller 304 is further configured to execute a body movement detecting program stored in the auxiliary storage part 309 or the like of each of the embodiments.

The main storage part 308 is a read-only memory (ROM) or a random access memory (RAM) that permanently or temporarily stores programs or data such as basic software of an operating system (OS) and application software, which are executed by the controller 304.

The auxiliary storage part 309 is a hard disk drive (HDD), which stores data associated with the application software.

A terminal interface part 305 is configured to perform a data adapter process and an interface process between a handset and external data terminals.

The microphone 306 is configured to convert sound into an electric signal. The microphone 306, for example, converts sound of a user during sleep into an acoustic signal.

The speaker 307 is configured to convert the electric signal into a physical vibration to generate sound such as music or voice.

Note that in the components of the body movement detecting apparatus illustrated according to the first and the second embodiments may be implemented by the body movement detecting program, which is executed by, for example, the controller 304 and the main storage part 308 serving as a working memory.

Accordingly, the mobile terminal apparatus 30 may be capable of detecting body movement of the user while the user is sleeping, which enables the mobile terminal apparatus 30 to automatically record a sleeping status of the user.

Further, the technologies discussed in the disclosures may be applied not only to the mobile terminal apparatus 30 but may also be applied to an information processing terminal having a built-in microphone or an external microphone to receive sound.

Moreover, a non-transitory recording medium may store the body movement detecting program that realizes a sequence of the body movement detecting processes described in the first and the second embodiments. Accordingly, the sequence of the body movement detecting processes may be implemented by a computer when the body movement detecting program stored in the non-transitory recording medium is executed by the computer. For example, the body movement detecting program may be stored in a recording medium, and hence the body movement detecting program may be read by a computer or the mobile terminal apparatus to implement the body movement detecting processes.

Note that various types of recording media may be used as the recording medium. Examples of the recording medium include a CD-ROM, a flexible disk and a magneto-optical disk on which information is optically, electrically or magnetically recorded; or a semiconductor memory such as a ROM or a flash memory on which information is electrically recorded.

According to the technologies disclosed in the first embodiment, body movement may be detected with high accuracy by utilizing the acoustic characteristics of the body movement sound.

The disclosed technologies are described according to the first and second embodiments; however, the disclosed technologies are not limited to the disclosed embodiments. Various modifications or alterations may be made within the scope of the invention described in the claims. Further, combinations of all or part of the components of aforementioned embodiments may be applied.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of the superiority or inferiority of the invention. Although the embodiments of the present invention have been described in detail, it should be understood that the various changes, substitutions, and alterations could be made hereto without departing from the scope of the invention as defined by the claims.

**Claims**

1. A body movement detecting apparatus (10, 20) comprising:

   a time-frequency converting unit (101) configured to convert an input acoustic signal to frequency component spectra;
   an electric power difference computing unit (103, 203) configured to compute an electric power difference value, between frequency component spectra taken at different times, for each of a plurality of predetermined frequency bandwidths;
   a duration computing unit (105);
   a similarity value computing unit (106, 201); and
   a body movement detecting unit (107, 202),
   **characterized in that**
   the duration computing unit (105) is configured to compute a duration in which a number of the predetermined frequency bandwidths each exhibiting the electric power difference value less than a first threshold;
   the similarity value computing unlit (106, 201) is configured to compute a similarity value indicating similarity of the electric power difference value of each of the predetermined frequency bandwidths;
   the body movement detecting unit (107, 202) is configured to detect that a body movement is present when the computed duration is equal to or greater than a second threshold and the computed similarity value is equal to or greater than a third threshold.

2. The body movement detecting apparatus as claimed in claim 1, wherein
   the similarity value computing unit (106, 201) is configured to compute the similarity value indicating the similarity of the electric power difference value of each of the predetermined frequency bandwidths when the duration is equal to or greater than a fourth threshold.

3. The body movement detecting apparatus as claimed in claim 1 or 2, wherein
   the similarity value computing unit (106, 201) is configured to compute the similarity value based on a sum of correlation values between the electric power difference values of the frequency bandwidths.

4. The body movement detecting apparatus as claimed in claim 1 or 2, wherein
   the similarity value computing unit (106, 201) is configured to compute the similarity value based on a value acquired by a signum function of the electric power difference value.

**5.** The body movement detecting apparatus as claimed in claim 4, wherein
the value acquired by the signum function is one of a number of the predetermined frequency bandwidths each exhibiting the electric power difference value equal to a positive value and a number of the predetermined frequency bandwidths each exhibiting the electric power difference value equal to 0 or a negative value that is greater than the other.

**6.** A body movement detecting method executed by a computer, the method comprising:

converting an input acoustic signal to frequency component spectra;
computing an electric power difference value, between frequency component spectra taken at different times, for each of a plurality of predetermined frequency bandwidths;
computing a duration in which a number of the predetermined frequency bandwidths each exhibiting the electric power difference value less than a first threshold;
computing a similarity value indicating similarity of the electric power difference value of each of the predetermined frequency bandwidths; and
detecting the presence of body movement based if the computed duration is equal to or greater than a second threshold and the computed similarity value is equal to or greater than a third threshold.

**7.** A body movement detecting computer program, which, when executed on an apparatus according to claim 1, is adapted to perform the steps of method claim 6.

**8.** A computer-readable storage medium storing the computer program of claim 7.

**Patentansprüche**

**1.** Körperbewegungserfassungsvorrichtung (10, 20), umfassend:

eine Zeit-Frequenz-Umwandlungseinheit (101), die so konfiguriert ist, dass sie ein Eingangsakustiksignal in Frequenzkomponentenspektren umwandelt;
eine Leistungsdifferenzberechnungseinheit (103, 203), die konfiguriert ist, um einen elektrischen Stromdifferenzwert zwischen den Frequenzkomponentenspektren, die zu verschiedenen Zeitpunkten genommen wurden, für jede einer Vielzahl von vorbestimmten Frequenzbandbreiten zu berechnen;
eine Dauerberechnungseinheit (105);
eine Ähnlichkeitswertberechnungseinheit (106, 201); und
eine Körperbewegungserfassungseinheit (107, 202),
**dadurch gekennzeichnet, dass**
die Dauerberechnungseinheit (105) konfiguriert ist, um eine Dauer zu berechnen, in der eine Anzahl der vorbestimmten Frequenzbandbreiten jeweils den elektrischen Leistungsdifferenzwert aufweist, der geringer ist als ein erster Schwellenwert;
die Ähnlichkeitswertberechnungseinheit (106, 201) konfiguriert ist, um einen Ähnlichkeitswert zu berechnen, der die Ähnlichkeit des elektrischen Leistungsdifferenzwerts von jeder der vorbestimmten Frequenzbandbreiten angibt;
die Körperbewegungserfassungseinheit (107, 202) konfiguriert ist, um zu erfassen, dass eine Körperbewegung vorhanden ist, wenn die berechnete Dauer gleich oder größer ist als ein zweiter Schwellenwert und wenn der berechnete Ähnlichkeitswert gleich oder größer ist als ein dritter Schwellenwert.

**2.** Körperbewegungserfassungsvorrichtung nach Anspruch 1, wobei
die Ähnlichkeitswertberechnungseinheit (106, 201) konfiguriert ist, um den Ähnlichkeitswert zu berechnen, der die Ähnlichkeit des elektrischen Leistungsdifferenzwerts von jeder der vorbestimmten Frequenzbandbreiten angibt, wenn die Dauer gleich oder größer ist als ein vierter Schwellenwert.

**3.** Körperbewegungserfassungsgerät nach Anspruch 1 oder 2, wobei
die Ähnlichkeitswertberechnungseinheit (106, 201) konfiguriert ist, um den Ähnlichkeitswert auf der Grundlage einer Summe von Korrelationswerten zwischen den elektrischen Leistungsdifferenzwerten der Frequenzbandbreiten zu berechnen.

**4.** Körperbewegungserfassungsvorrichtung nach Anspruch 1 oder 2, wobei

die Ähnlichkeitswertberechnungseinheit (106, 201) konfiguriert ist, um den Ähnlichkeitswert auf der Grundlage eines Wertes zu berechnen, der durch eine Vorzeichenfunktion des elektrischen Leistungsdifferenzwerts erfasst wird.

**5.** Körperbewegungserfassungsvorrichtung nach Anspruch 4, wobei

der durch die Vorzeichenfunktion erfasste Wert einer von einer Anzahl der vorbestimmten Frequenzbandbreiten ist, die jeweils den elektrischen Leistungsdifferenzwert gleich einem positiven Wert und einer Anzahl der vorbestimmten Frequenzbandbreiten aufweisen, die jeweils den elektrischen Leistungsdifferenzwert gleich 0 oder einen negativen Wert aufweisen, der größer ist als der andere.

**6.** Körperbewegungserfassungsverfahren, das von einem Computer ausgeführt wird, wobei das Verfahren umfasst:

Umwandeln eines akustischen Eingangssignals in Frequenzkomponentenspektren;
Berechnen eines elektrischen Leistungsdifferenzwerts zwischen Frequenzkomponentenspektren, die zu verschiedenen Zeitpunkten aufgenommen wurden, für jede einer Vielzahl von vorbestimmten Frequenzbandbreiten;
Berechnen einer Dauer, in welcher eine Anzahl von vorbestimmten Frequenzbandbreiten jeweils einen elektrischen Leistungsdifferenzwert zeigen, der kleiner ist als ein erster Schwellenwert;
Berechnen eines Ähnlichkeitswertes, der eine Ähnlichkeit des elektrischen Leistungsdifferenzwerts jeder der vorbestimmten Frequenzbandbreiten anzeigt; und
Erfassen des Vorhandenseins von Körperbewegungen, wenn die berechnete Dauer gleich oder größer ist als ein zweiter Schwellenwert und der berechnete Ähnlichkeitswert gleich oder größer ist als ein dritter Schwellenwert.

**7.** Körperbewegungserfassungs-Computerprogramm, welches, wenn es auf einer Vorrichtung nach Anspruch 1 ausgeführt wird, geeignet ist, die Schritte des Verfahrensanspruchs 6 durchzuführen.

**8.** Ein Computer-lesbares Speichermedium, welches das Programm nach Anspruch 7 speichert.


**Revendications**

**1.** Appareil de détection de mouvement de corps (10, 20) comprenant :

une unité de conversion temps-fréquence (101) configurée pour convertir un signal acoustique d'entrée en des spectres de composantes de fréquence ;
une unité de calcul de différence de puissance électrique (103, 203) configurée pour calculer une valeur de différence de puissance électrique, entre des spectres de composantes de fréquence pris à des instants différents, pour chacune d'une pluralité de largeurs de bande de fréquence prédéterminées ;
une unité de calcul de durée (105) ;
une unité de calcul de valeur de similitude (106, 201) ; et
une unité de détection de mouvement de corps (107, 202),
**caractérisé en ce que**
l'unité de calcul de durée (105) est configurée pour calculer une durée pendant laquelle un nombre des largeurs de bande de fréquence prédéterminées présentent chacune la valeur de différence de puissance électrique inférieure à un premier seuil ;
l'unité de calcul de valeur de similitude (106, 201) est configurée pour calculer une valeur de similitude indiquant une similitude de la valeur de différence de puissance électrique de chacun des largeurs de bande de fréquence prédéterminées ;
l'unité de détection de mouvement de corps (107, 202) est configurée pour détecter qu'un corps est présent lorsque la durée calculée est égale ou supérieure à un deuxième seuil et la valeur de similitude calculée est égale ou supérieure à un troisième seuil.

**2.** Appareil de détection de mouvement de corps selon la revendication 1, dans lequel
l'unité de calcul de valeur de similitude (106, 201) est configurée pour calculer la valeur de similitude indiquant la similitude de la valeur de différence de puissance électrique de chacune des largeurs de bande de fréquence prédéterminées lorsque la durée est égale ou supérieure à un quatrième seuil.

**3.** Appareil de détection de mouvement de corps selon la revendication 1 ou 2, dans lequel

l'unité de calcul de valeur de similitude (106, 201) est configurée pour calculer la valeur de similitude sur la base d'une somme de valeurs de corrélation entre les valeurs de différence de puissance électrique des largeurs de bande de fréquence.

4. Appareil de détection de mouvement de corps selon la revendication 1 ou 2, dans lequel
l'unité de calcul de valeur de similitude (106, 201) est configurée pour calculer la valeur de similitude sur la base d'une valeur acquise par une fonction signe de la valeur de différence de puissance électrique.

5. Appareil de détection de mouvement de corps selon la revendication 4, dans lequel
la valeur acquise par la fonction signe est l'un d'un nombre des largeurs de bande de fréquence prédéterminées présentant chacune la valeur de différence de puissance électrique égale à une valeur positive, et d'un nombre des largeurs de bande de fréquence prédéterminées présentant chacune la valeur de différence de puissance électrique égale à 0 ou une valeur négative qui est supérieure à l'autre.

6. Procédé de détection de mouvement de corps exécuté par un ordinateur, le procédé comprenant les étapes consistant à :

convertir un signal acoustique d'entrée en spectres de composantes de fréquence ;
calculer une valeur de différence de puissance électrique, entre des spectres de composantes de fréquence pris à des instants différents, pour chacune d'une pluralité de largeurs de bande de fréquence prédéterminées ;
calculer une durée dans laquelle un nombre des largeurs de bande de fréquence prédéterminées présentent chacune la valeur de différence de puissance électrique inférieure à un premier seuil ;
calculer une valeur de similitude indiquant une similitude de la valeur de différence de puissance électrique de chacune des largeurs de bande de fréquence prédéterminées ; et
détecter la présence de mouvement de corps repose en se basant sur la durée calculée qui est égale ou supérieure à un deuxième seuil et la valeur de similitude calculée qui est égale ou supérieure à un troisième seuil.

7. Programme informatique de détection de mouvement de corps qui, lorsqu'il est exécuté sur un appareil selon la revendication 1, est adapté pour exécuter les étapes du procédé de la revendication 6.

8. Support de stockage lisible par ordinateur stockant le programme informatique selon la revendication 7.

FIG.1A

FIG.1B

FIG.1C

# FIG.2

# FIG.3

ELECTRIC POWER
DIFFERENCE

FIG.4A

20
10
0
−10
−20

TIME

ELECTRIC POWER
DIFFERENCE

FIG.4B

20
10
0
−10
−20

TIME

# FIG.5

ELECTRIC POWER
DIFFERENCE

---------- ELECTRIC POWER DIFFERENCE IN 5 kHZ BANDWIDTH
——————— ELECTRIC POWER DIFFERENCE IN 7 kHZ BANDWIDTH

FIG.6A

20
10
0
-10
-20

TIME

ELECTRIC POWER
DIFFERENCE

---------- ELECTRIC POWER DIFFERENCE IN 5 kHZ BANDWIDTH
——————— ELECTRIC POWER DIFFERENCE IN 7 kHZ BANDWIDTH

FIG.6B

20
10
0
-10
-20

TIME

NUMBER OF FREQUENCY BANDWIDTHS

THRESHOLD 1

(A)

TIME

CORRELATION

FIG.7

THRESHOLD 2

(B)

TIME

BODY MOVEMENT DETECTED

NO BODY MOVEMENT DETECTED

(C)

TIME

W1

# FIG.8

**FIG.9**

NUMBER OF FREQUENCY BANDWIDTHS

THRESHOLD 1

W2

TIME

(A)

CORRELATION

THRESHOLD 2

TIME

(B)

BODY MOVEMENT DETECTED

NO BODY MOVEMENT DETECTED

TIME

(C)

# FIG.10

**FIG.11**

NUMBER OF FREQUENCY BANDWIDTHS

W3

THRESHOLD 1

TIME

(A)

CORRELATION

THRESHOLD 2

TIME

(B)

BODY MOVEMENT DETECTED

NO BODY MOVEMENT DETECTED

(C)

TIME

**FIG.12**

START

PERFORM
TIME-FREQUENCY
CONVERSION
S101

COMPUTE
ELECTRIC POWER
S102

COMPUTE
ELECTRIC POWER
DIFFERENCE
S103

COMPUTE
CORRELATION
BETWEEN ELECTRIC
POWER DIFFERENCES
S104

S105
NUMBER
OF FREQUENCY
BANDWIDTHS EACH
EXHIBITING ELECTRIC POWER
DIFFERENCE < TH_POW IS
EQUAL TO OR GREATER
THAN TH_NUM
?

YES

S106
ADD 1
FRAME TO
DURATION

NO

S107
DURATION
TO PREVIOUS FRAME
IS EQUAL TO OR GREATER
THAN TH_TIME
?

NO

YES

S109
SUM OF
CORRELATION VALUES
IS EQUAL TO OR GREATER
THAN TH_COR
?

NO

S108
DETERMINE
THAT
DURATION
INCLUDES
NO BODY
MOVEMENT

YES

S110
DETERMINE THAT
DURATION INCLUDES
BODY MOVEMENT

END

EP 2 526 864 B1

FIG.13

ACOUSTIC
SIGNAL →
┌─────────────────────────────────────────────────────────────────────────────────┐ 20
│  101        102        103        105        201        202                        │
│ ┌────────┐ ┌────────┐ ┌────────┐ ┌────────┐ ┌────────┐ ┌────────┐                 │
│ │ TIME-  │ │ELECTRIC│ │ELECTRIC│ │        │ │SIMILAR-│ │ BODY   │                 │
│ │FREQUEN-│ │ POWER  │ │ POWER  │ │DURATION│ │ ITY    │ │MOVEMENT│                 │
│ │ CY     │→│COMPUT- │→│DIFFER- │→│COMPUT- │→│ VALUE  │→│DETECT- │→ DETECTED       │
│ │CONVERT-│ │ING     │ │ENCE    │ │ING     │ │COMPUT- │ │ING     │    RESULT       │
│ │ING     │ │PART    │ │COMPUT- │ │PART    │ │ING     │ │PART    │                 │
│ │PART    │ │        │ │ING     │ │        │ │PART    │ │        │                 │
│ └────────┘ └────────┘ │PART    │ └────────┘ └────────┘ └────────┘                 │
│                       └────┬───┘                 ↑                                 │
│                            ↓      104            │                                 │
│                         ┌──────┐                 │                                 │
│                         │STORAGE│────────────────┘                                 │
│                         │PART  │                                                   │
│                         └──────┘                                                   │
└─────────────────────────────────────────────────────────────────────────────────┘

29

# FIG.14

DURATION TO PREVIOUS FRAME ($\geq$ TH_TIME)

COMPUTE CORRELATION IN THIS DURATION    CURRENT FRAME

**FIG.15**

```
         ┌─────────────┐
         │    START    │
         └──────┬──────┘
                │
    ┌───────────▼───────────┐
    │       PERFORM         │  ──S201
    │   TIME-FREQUENCY      │
    │     CONVERSION        │
    └───────────┬───────────┘
                │
    ┌───────────▼───────────┐
    │      COMPUTE          │  ──S202
    │   ELECTRIC POWER      │
    └───────────┬───────────┘
                │
    ┌───────────▼───────────┐
    │      COMPUTE          │  ──S203
    │   ELECTRIC POWER      │
    │     DIFFERENCE        │
    └───────────┬───────────┘
                │
```

S204

NUMBER
OF FREQUENCY
BANDWIDTHS EACH
EXHIBITING ELECTRIC POWER
DIFFERENCE < TH_POW IS
EQUAL TO OR GREATER
THAN TH_NUM
?

YES

NO

S205

ADD 1
FRAME TO
DURATION

S206

DURATION
TO PREVIOUS FRAME
IS EQUAL TO OR GREATER
THAN TH_TIME
?

NO

YES

S208

COMPUTE
CORRELATION
BETWEEN ELECTRIC
POWER DIFFERENCES

S209

SUM OF
CORRELATION VALUES
IS EQUAL TO OR GREATER
THAN TH_COR
?

NO

YES

S210

DETERMINE THAT
DURATION INCLUDES
BODY MOVEMENT

S207

DETERMINE
THAT
DURATION
INCLUDES
NO BODY
MOVEMENT

```
         ┌─────────────┐
         │     END     │
         └─────────────┘
```

31

# FIG.16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007289660 A
- JP 2008301951 A
- WO 2011010384 A1
- US 2011112442 A1
- WO 2006033104 A1
- WO 2008098943 A2

**Non-patent literature cited in the description**

- **NAKAYAMA, E. et al.** A Basic Study on Sleep-Wake Identification by Wrist Actigraph. *Ishikawa Journal of Nursing,* 2006, vol. 3 (2), 31-37
- **KUNIEDA, M.** Practical use, Mechanical Vibration. Rikogakusha Publishing Co., Ltd, 1990